(19) Europäisches Patentamt
European Patent Office
Office européen des brevets

(11) Publication number: **0 074 272**
**B1**

(12) # EUROPEAN PATENT SPECIFICATION

(45) Date of publication of patent specification: **13.08.86**

(51) Int. Cl.$^4$: **C 07 C 45/00,** C 07 C 47/565

(21) Application number: **82304687.5**

(22) Date of filing: **07.09.82**

(54) **Process for the preparation of hydroxybenzaldehydes.**

(30) Priority: **07.09.81 JP 141484/81**

(43) Date of publication of application:
**16.03.83 Bulletin 83/11**

(45) Publication of the grant of the patent:
**13.08.86 Bulletin 86/33**

(84) Designated Contracting States:
**CH DE FR GB IT LI NL**

(56) References cited:
**FR-A-2 196 200**
**GB-A-1 490 350**
**US-A-4 324 922**

**TETRAHEDRON LETTERS, no. 39, 1979, pages 3753-3756, Pergamon Press Ltd., (GB); YOEL SASSON et al.: "The effect of phase transfer catalysts on the reimer-tiemann reaction"**

**The file contains technical information submitted after the application was filed and not included in this specification**

(73) Proprietor: **SUMITOMO CHEMICAL COMPANY, LIMITED**
**15 Kitahama 5-chome Higashi-ku**
**Osaka-shi Osaka 541 (JP)**

(72) Inventor: **Hamada, Kazuhiko**
**No. 2-1-248, Kuwatacho**
**Ibarakishi Osaka (JP)**
Inventor: **Suzukamo, Gohfu**
**No. 2-1-216, Kuwatacho**
**Ibarakishi Osaka (JP)**
Inventor: **Masuko, Fujio**
**No. 1-29-111 Kentoku-machi 3-chome**
**Oita-shi Oita (JP)**
Inventor: **Nakamura, Makoto**
**No. 2-1-138, Kuwatacho**
**Ibarakishi Osaka (JP)**

(74) Representative: **Moore, Anthony John et al**
**Gee & Co. Chancery House Chancery Lane**
**London WC2A 1QU (GB)**

**EP 0 074 272 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to a process for the preparation of hydroxybenzaldehydes and is an improvement of the Reimer-Tiemann reaction (i.e., the reaction of an alkaline solution of phenol with chloroform to prepare a mixture of salicylaldehyde (*o*-hydroxybenzaldehyde) and *p*-hydroxybenzaldehyde) in which the reaction is carried out in the presence of a phase transfer catalyst.

Of the hydroxybenzaldehydes obtainable by the process of the present invention, salicylaldehyde and *p*-hydroxybenzaldehyde are both industrially important compounds, the former being useful as an intermediate for the preparation of perfumes, agricultural chemicals and chelating agents, and the latter as an intermediate for the preparation of agricultural chemicals and medicines, e.g. penicillin modifiers.

The following methods are among those so far known for the preparation of hydroxybenzaldehydes:

(1) The Conventional Reimer-Tiemann Reaction:

In this reaction, an aqueous alkaline solution of a phenol is reacted with chloroform in a physically heterogeneous system to prepare a mixture of salicylaldehyde and *p*-hydroxybenzaldehyde. However, in this method, the yield of salicylaldehyde is generally low. Furthermore, an excess amount of chloroform with respect to the phenol is necessary, and the recovery and recycling of unreacted chloroform and phenol are difficult.

A typical example of an improved Reimer-Tiemann reaction is that in which aqueous methanol (containing from 10 to 75% by weight of methanol) is employed as a reaction medium (see U.S. Patent 3,365,500). Although this method has the advantage that the amount of tar formed is reduced, it has the defects that the conversion of phenol is low; the separation and recovery of unreacted phenol are difficult; and the yield ratio of salicylaldehyde to *p*-hydroxybenzaldehyde is lower than that obtained under the ordinary Reimer-Tiemann reaction conditions and there are difficulties in the separation of the components from the reaction mixture.

(2) Method proposed by Yoel Sasson *et al* [*Tetrahydron Lett.,* 3753 (1979) and 1875 (1980)]:

In accordance with this method, when an aliphatic tertiary amine is added to the reaction mixture obtained under the ordinary Reimer-Tiemann condition (where excess amounts of chloroform and an aqueous alkali solution are used with respect to the phenol), the yield of salicylaldehyde is improved without any undesirable effect on the yield of *p*-hydroxybenzaldehyde. However, this method is accompanied by several disadvantages: Firstly, the tertiary amines which can be used are limited to a few species such as $(n\text{-}C_4H_9)_3N$, and a competitive reaction to form an alkyl ether of the phenol also takes place. Secondly, excess amounts of chloroform and aqueous alkali solution with respect to the phenol need be used, which leads to the foregoing disadvantages; and thirdly, we have experimentally confirmed that, under the same conditions as reported, the effects brought about by the addition of the aliphatic tertiary amine are not as dramatic as reported.

(3) A method in which salicylaldehyde is prepared in good yield by one step reaction using as starting materials a phenol and formaldehyde and a specific catalyst (a base represented by a tertiary amine and/or an organometallic salt):

This method is described in the published unexamined Japanese Patent Applications (OPI) Nos. 34737/78 and 163538/79 and *J.C.S.,* Perkin I, 1862 (1980). This method, however, also has disadvantages: an excess amount of formaldehyde over the phenol, a poisonous catalyst (e.g., $SnCl_2$, $SnCl_4$ or $Cr(acac)_3$ or a fairly large amount of an organic amine as an additive needs be used, and therefore, a complicated posttreatment and waste water treatment and the like are needed.

(4) A method for selectively obtaining *p*-hydroxybenzaldehyde by oxidizing *p*-cresol derivatives with oxygen:

This method includes, for example, a method using an excessive amount of potassium tertiary butoxide in dimethylformamide (as described in *Angew. Chem.,* 86, 386 (1975)). Such a method, however, requires that large amounts of a special solvent and base be used, and the yield of desired product is very low when a starting material other than 2,6-disubstituted *p*-cresol is used.

We have discovered that when the Reimer-Tiemann reaction is carried out in the presence of a phase transfer catalyst, the reaction rate, the overall yield and yield ratio of salicylaldehyde/*p*-hydroxybenzaldehyde, the percentage of unreacted phenol recovered and the like can be controlled and improved depending upon the kind of phase transfer catalyst, the kind and concentration of the aqueous alkali solution, the kind of the organic solvent and the ratio of the reaction reagents employed.

Commercially-available phenol, chloroform and alkali metal hydroxide can be used as they are as reagents in the process of the present invention.

GB Specification No. 1490350 describes the manufacture of a 2-hydroxy naphthaldehyde by reacting a 2-naphthol alkaline metal salt with chloroform in aqueous solution, e.g. in sodium hydroxide solution, in the presence of a phase transfer catalyst, preferably a quaternary ammonium salt; chloroform is dropped into the aqueous solution so that the reaction takes place in a heterogeneous system. The process relates only to treatment of a 2-hydroxy naphthaldehyde starting material.

French Specification No. 2196200 describes at page 26 a process for the preparation of 2-hydroxy 5-butylbenzaldehyde, by reacting 4-butylphenol with chloroform in the presence of sodium hydroxide solution, with triethylbenzylammonium chloride which serves as a catalyst.

Both these prior disclosures relates to the use of a starting material which is substituted in the aromatic ring (other than with a hydroxy group).

We have now discovered that when the reaction of the aforesaid type, using a phase transfer catalyst, is carried out using an unsubstituted phenolic starting material and a specific combination of catalyst and solvent, both ortho- and para- isomers can be synthesized in good yield, and without substantial formation of by-products and with high recovery of unreacted phenol starting material.

Thus, according to the invention we provide a process for the preparation of salicylaldehyde and *p*-hydroxybenzaldehyde which comprises reacting an unsubstituted phenolic compound in aqueous alkali solution with chloroform in a heterogeneous system according to the Reimer-Tiemann reaction, in the presence of a phase transfer catalyst, characterized in that the phase transfer catalyst is a cationic-type phase transfer catalyst used in the presence of an aliphatic noncyclic ether as solvent or an amphoteric-type phase transfer catalyst used in the presence of an inert organic solvent.

Commercially available phenol, chloroform and alkali metal hydroxide can be used as they are as reagents in the process of the present invention.

Examples of alkali metal hydroxides which can be used include lithium hydroxide, sodium hydroxide, potassium hydroxide and cesium hydroxide, sodium hydroxide and potassium hydroxide being preferable because they are inexpensive. The concentration of the aqueous solution of alkali metal hydroxide which can be used is usually from 10 to 60%. In general, the amount of salicylaldehyde formed increases and the amount of *p*-hydroxybenzaldehyde formed decreases, as the concentration of alkali metal hydroxide in the aqueous solution increases.

The formation ratio of the two hydroxybenzaldehydes (e.g., salicylaldehyde and *p*-hydroxybenzaldehyde) varies depending upon the kind of alkali metal hydroxide used. For example, with a 50% aqueous solution of a hydroxide the formation ratio of salicylaldehyde to *p*-hydroxybenzaldehyde decreases when using the specific compounds lithium hydroxide, sodium hydroxide, potassium hydroxide and cesium hydroxide in that order. In other words, the formation ratio is highest when using lithium hydroxide, somewhat lower when using sodium hydroxide, still lower when using potassium hydroxide, and lowest when using cesium hydroxide.

Examples of the organic solvent inert to the reaction which can optionally be used in the present invention include aromatic and aromatic halogenated hydrocarbons such as benzene, toluene, xylene and chlorobenzene; ethers such as anisole, diisopropyl ether, dibutyl ether and tetrahydrofuran; aliphatic alcohols such as methanol, ethanol and butanol; esters such as ethyl acetate and methyl acetate; ketones such as methyl isobutyl ketone; aliphatic hydrocarbons such as *n*-hexane and cyclohexane; and heterogenous polar solvents such as acetonitrile, dimethyl sulfoxide and dimethylformamide.

With solvents forming a heterogeneous reaction system such as aromatic hydrocarbons, aliphatic hydrocarbons and ethers, not only is the recovery percentage of phenol high but also the yield of hydroxybenzaldehydes. On the other hand, with solvents forming a homogeneous reaction system such as polar solvents and cyclic ethers, the recovery percentage of phenol is somewhat lower. The solvents affording the best results (i.e., highest yield based on phenol consumed) are aliphatic non cyclic ether solvents such as dibutyl ether and diisopropyl ether. The amount of the inert organic solvent used is from 0.5 to 10 parts by weight, preferably 1 to 5 parts by weight, based on the phenol as the starting material.

Examples of the phase transfer catalyst which can be used in the reaction of the present invention include cationic-type (quarternary salt-type), amphoteric-type and anionic-type, the cationic and amphoteric types being most effective. Examples of suitable cationic-type phase transfer catalysts include quaternary ammonium salts and quaternary phosphonium salts such as cetyltrimethylammonium chloride, cetyltributylphosphonium bromide, triethylbenzylammonium chloride and tetrabutylammonium hydroxide, and pyridinium salts. Those which are particularly preferable for the preparation of salicylaldehyde are cationic-type quaternary salts in which the anionic moiety thereof consists of a hydroxy group. Examples of suitable amphoteric-type phase transfer catalysts include those of the betaine-type and amino acid-type, and those which are particularly preferable for the preparation of *p*-hydroxybenzaldehyde are betaine-type amphoteric compounds such as lauryl betaine. Examples of suitable anionic-type phase transfer catalyst include alkali metal salts of higher fatty acids, higher alkylsulfonates and salts of sulfuric acid esters of higher alcohols.

The cationic-type phase transfer catalyst acts as follows: when it is used in an alkali solution having a high concentration, e.g. from 40 to 60%, the percentage of unreacted phenol recovered generally increases (e.g. to 89 to 95%), and at the same time, with an increase in the concentration of alkali metal hydroxide in the aqueous solution (e.g., from 10% to 60%), the amount mainly of salicylaldehyde among the hydroxybenzaldehydes formed increases. Furthermore, it is observed that the reaction rate also increases and is generally from 1.5 to 4 times as high as that when using no catalyst. Also, when the amphoteric-type phase transfer catalyst is used in an aqueous solution system having a low concentration, the amount of *p*-hydroxybenzaldehyde increases. A catalytic amount of the phase transfer catalyst with respect to the chloroform is used, for example, from 0.1 to 20 mole %, preferably from 0.5 to 5 mole %, based on the chloroform.

With respect to the feed ratio of the reaction reagents, there can be employed various conditions ranging from normal experimental conditions in which the aqueous alkali solution and chloroform are used in large excess with respect to the phenol (for example, phenol/alkali/chloroform = 1.0/10.0/10.0) to

conditions in which the alkali and chloroform are used in amounts below the assumed theoretical amounts (for example, phenol/alkali/chloroform = 1.0/4.0/1.0). The range of feed ratios of phenol/alkali/chloroform can thus be stated as from 1.0/10.0/10.0 to 1.0/2.0/0.33. When the amount of chloroform is large, the amount of tar, which is a high-boiling substance, increases and hence it is desirable to employ a method in which the amount of chloroform is decreased and the amount of alkali is somewhat increased. An optimum feed ratio of reaction reagents is, for example, a ratio of phenol/alkali (sodium hydroxide)/chloroform of 1.5/5.4/1.0.

With respect to the separation and purification of the reaction products, we have found that high-purity hydroxybenzaldehydes (i.e., salicylaldehyde and *p*-hydroxybenzaldehyde) can be obtained in good efficiency, for example, by a method outlined below:

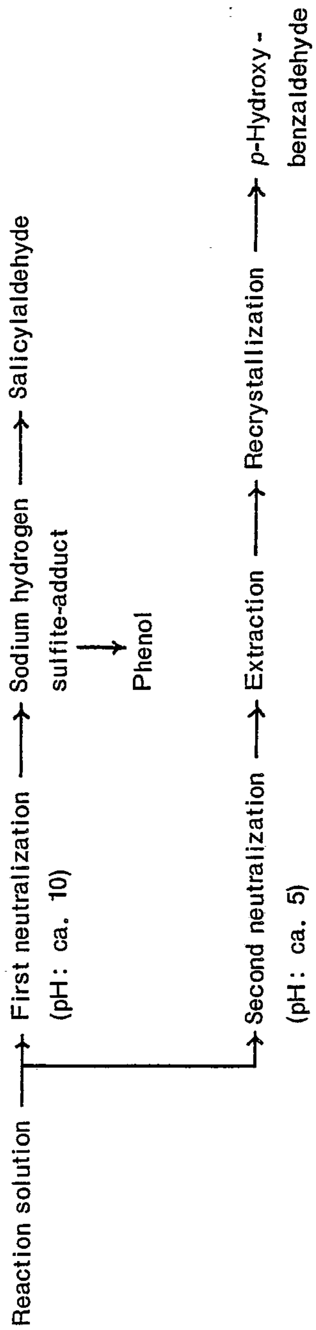

Reaction solution
First neutralization
(pH: ca. 10)
Sodium hydrogen
sulfite-adduct
Phenol
Salicylaldehyde
Second neutralization
(pH: ca. 5)
Extraction
Recrystallization
*p*-Hydroxy-
benzaldehyde

The separation and purification of these reaction products can be carried out by the foregoing method to obtain two kinds of high-purity hydroxybenzaldehydes with high efficiency.

The analysis of the products of the process of the present invention (unreacted phenol, hydroxybenzaldehydes and high-boiling substances) was principally carried out according to gas chromatographic quantitative analysis using an internal standard. By this analysis a main component of the high-boiling substances formed in small amounts by the reaction of the present invention was identified as triphenoxymethane

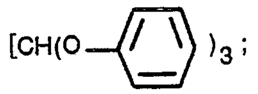

molecular weight, 292]. Two kinds of dialdehydes having the structural formulae (1) and (2) set forth below are produced in trace amounts as by-products; they have not, however, so far been confirmed among the products of the ordinary Reimer-Tiemann reaction.

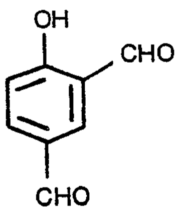

(1)

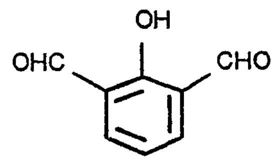

(2)

We are the first to identify the dialdehyde (1) by isolation, gas chromatographic analysis, measurement of physical property and synthesis through another route. The dialdehyde (2) was confirmed by mass spectral analysis.

The present invention will now be illustrated by the following Examples, in which percentages are given by weight.

## Example 1

A 50% sodium hydroxide aqueous solution comprising 86.4 g (2.16 mole) of commercially-available special-grade sodium hydroxide (purity: 96.0%) and 86.4 g (4.80 mole) of water was fed into a three-necked flask equipped with a condenser, and then 56.4 g (0.6 mole) of phenol was added thereto to form a salt. Thereafter, 70 ml of di-*n*-butyl ether and 1.3 g of tetrabutylammonium hydroxide (40% methanol solution, 0.002 mole) were added thereto, and the resulting mixture was heated to 55° to 57°C while slowly stirring using a stirring blade. After confirming that the temperature of the reaction system had become constant and stirring could be conducted smoothly (200 to 300 rpm), 47.7 g (0.4 mole) of chloroform held in a side-tube was slowly and carefully added dropwise to the stirred solution over about 1 hour. After completion of the dropwise addition, the reaction system was kept at the same temperature with stirring for about 2.5 hours. During that period, cooling by means of a condenser supplied with ice water had to be carried out in order to provide sufficient cooling to prevent chloroform from escaping from the system. After completion of the period of temperature maintenance, the reaction system was cooled to 20°C and hydrolysed with a 10% hydrochloric acid aqueous solution (pH: about 5).

After separating the organic layer, the aqueous layer was extracted twice with 40 ml of ethyl acetate (or methyl isobutyl ketone). An organic layer obtained by combining these extracts and the aqueous layer were quantitatively analyzed by gas chromatography in accordance with a GC—IS method. The results obtained are shown in Table 1.

TABLE 1

| Recovery Percentage of Unreacted Phenol | Product Yield per Phenol Consumed (%) | | | |
|---|---|---|---|---|
| (%) | SAL *1 | POBA *2 | SAL + POBA | SAL/POBA |
| 94.8 | 70.5 | 15.2 | 85.7 | 4.6 |

Note *1 Salicylaldehyde

Note *2 *p*-Hydroxybenzaldehyde

Examples 2 to 19

Reactions were carried out in the same manner as in Example 1. The results obtained are shown in Table 2. SAL and POBA in Table 2 have the same meanings as in Table 1.

Examples 20 to 22

Reactions were carried out in the same manner as in Example 1. The results obtained are shown in Table 3. SAL and POBA in Table 2 again have the same meanings as in Table 1.

TABLE 2

| Example No. | Feeding: Phenol (mole) | Feeding: Chloroform (mole) | Feeding: Alkali (kind) (mole) | Feeding: Alkali (kind) (aqueous solution, %) | Feeding: Phase Transfer Catalyst (mole) | Feeding: Inert Organic Solvent (ml) | Reaction Condition: Temperature (°C) | Reaction Condition: Time (hr) | Recovery Percentage of Unreacted Phenol (%) | Product (Yield per Phenol Consumed) (%): SAL | POBA | SAL + POBA | SAL/POBA |
|---|---|---|---|---|---|---|---|---|---|---|---|---|---|
| 2 | 0.6 | 0.2 | NaOH 1.2 | (50%) | BAC *1 0.002 | *n*-Butyl ether 60 | 55 Chloroform added dropwise | 3.5 | 94.1 | 61.2 | 14.0 | 75.2 | 4.4 |
| 3 | 0.6 | 0.2 | NaOH 1.2 | (50%) | BAC 0.002 | Anisole 60 | 55 Same as above | 3.5 | 91.7 | 45.2 | 9.6 | 54.8 | 4.7 |
| 4 | 0.6 | 0.2 | NaOH 0.6 | (50%) | BAC 0.002 | Toluene 60 | 55 aq. NaOH and chloroform added dropwise simultaneously | 3.5 | 89.9 | 45.6 | 7.9 | 53.5 | 5.8 |
| 5 | 0.6 | 0.2 | NaOH 1.2 | (50%) | BAC 0.002 | Toluene 120 | 55 Chloroform added dropwise | 3.5 | 90.5 | 39.3 | 12.5 | 51.8 | 3.14 |
| 6 (Comparative Example) | 0.6 | 0.2 | NaOH 1.2 | (50%) | None | Toluene 120 | 55 Same as above | 5.0 | 81.2 | 32.5 | 10.5 | 43.0 | 3.09 |
| 7 | 0.6 | 0.2 | NaOH 1.2 | (70%) | BAC 0.002 | None | 55 Chloroform added dropwise | 3.5 | 80.0 | 43.2 | 5.8 | 49.0 | 7.4 |
| 8 | 0.6 | 0.2 | NaOH 1.2 | (40%) | BAC 0.002 | None | Same as above | | 84.6 | 36.9 | 16.1 | 53.0 | 2.3 |

Note *1 $(C_2H_5)_3C_6H_5CH_2NCl$

TABLE 2 (Continued)

| Example No. | Feeding: Phenol | Feeding: Chloroform | Feeding: Alkali (kind) | | Feeding: Phase Transfer Catalyst | Feeding: Inert Organic Solvent | Reaction Condition: Temperature | Reaction Condition: Time | Recovery Percentage of Unreacted Phenol | Product (Yield per Phenol Consumed) (%) | | | |
|---|---|---|---|---|---|---|---|---|---|---|---|---|---|
| | (mole) | (mole) | (mole) | (aqueous solution, %) | (mole) | (ml) | (°C) | (hr) | (%) | SAL | POBA | SAL + POBA | SAL/POBA |
| 9 | 0.6 | 1.2 | NaOH 2.4 | (10%) | Lauryl *2 betaine 0.006 | Benzene 100 | 75 Chloroform added dropwise | 4.0 | 78.3 | 17.1 | 43.9 | 61.0 | 0.4 |
| 10 | 0.6 | 1.2 | NaOH 2.4 | (10%) | Glycine type *3 0.006 | Benzene 100 | 75 Same as above | 5.0 | 79.2 | 28.9 | 24.1 | 53.0 | 1.2 |
| 11 | 0.6 | 0.2 | NaOH 1.2 | (50%) | BAC 0.001 | None | 55 Same as above | 3.5 | 85.0 | 43.6 | 12.8 | 56.4 | 3.4 |
| 12 | 0.2 | 0.6 | NaOH 2.0 | (50%) | BAC 0.001 | None | Same as above | | 36.7 | 40.9 | 11.1 | 52.0 | 3.7 |
| 13 | 0.6 | 0.2 | NaOH 1.2 | (50%) | BAC 0.002 | *n*-Hexane | Same as above | | 87.7 | 44.0 | 13.2 | 57.2 | 3.3 |
| 14 | 0.6 | 0.2 | NaOH 1.2 | (50%) | $(C_4H_9)_4NOH$ 0.002 | Xylene 60 | Same as above | | 91.3 | 49.1 | 12.9 | 62.0 | 3.8 |
| 15 | 0.6 | 0.2 | NaOH 1.2 | (50%) | $(C_4H_9)_4NOH$ 0.002 | Isopropyl ether 60 | Same as above | | 92.1 | 51.2 | 12.0 | 63.2 | 4.3 |

Note *2 $CH_3(CH_2)_{11}\overset{\oplus}{N}(CH_3)_2CH_2COO^{\ominus}$
*3 $(C_8H_{17})_2NCH_2CO_2H$

TABLE 2 (Continued)

| Example No. | Feeding: Phenol | Feeding: Chloroform | Feeding: Alkali (kind) | | Feeding: Phase Transfer Catalyst | Feeding: Inert Organic Solvent | Reaction Condition: Temperature | Reaction Condition: Time | Recovery Percentage of Unreacted Phenol | Product (Yield per Phenol Consumed) (%) | | | |
|---|---|---|---|---|---|---|---|---|---|---|---|---|---|
| | (mole) | (mole) | (mole) | (aqueous solution, %) | (mole) | (ml) | (°C) | (hr) | (%) | SAL | POBA | SAL + POBA | SAL/POBA |
| 16 | 0.6 | 0.2 | NaOH 1.2 | (50%) | $C_{12}H_{25}C_6H_4SO_3Na$ 0.002 | Methanol 30 | 55 Chloroform added dropwise | 3.5 | 84.0 | 34.3 | 14.8 | 49.1 | 2.3 |
| 17 | 0.6 | 0.2 | NaOH 1.2 | (50%) | $C_{16}H_{33}N(CH_3)_3Cl$ 0.002 | Aceto-nitrile 30 | Same as above | | 87.5 | 34.7 | 25.0 | 59.7 | 1.4 |
| 18 | 0.6 | 0.4 | NaOH 2.16 | (50%) | $C_{12}H_{25}N(CH_3)_3Cl$ 0.002 | *n*-Butylether 70 | Same as above | | 93.5 | 63.7 | 13.9 | 77.6 | 4.6 |
| 19 | 0.6 | 0.4 | NaOH 2.16 | (50%) | $[(CH_3)_3NCH_2C_6H_5]OH$ 0.002 | Isopropyl-ether 70 | Same as above | | 91.1 | 53.7 | 13.1 | 66.8 | 4.1 |

TABLE 3

| Example No. | Feeding: Phenol | Feeding: Chloroform | Feeding: Alkali (kind) | | Feeding: Phase Transfer Catalyst | Feeding: Inert Organic Solvent | Reaction Condition: Temperature | Reaction Condition: Time | Recovery Percentage of Unreacted Phenol | Product (Yield per Phenol Consumed) (%) | | | |
|---|---|---|---|---|---|---|---|---|---|---|---|---|---|
| | (mole) | (mole) | (mole) | (aqueous solution, %) | (mole) | (ml) | (°C) | (hr) | (%) | SAL | POBA | SAL + POBA | SAL/POBA |
| 20 | 0.6 | 0.2 | KOH 1.2 | (50%) | $(C_4H_9)_4NOH$ 0.003 | *n*-Butyl ether 60 | 60 Chloroform added dropwise | 4.5 | 90.2 | 37.7 | 23.6 | 61.3 | 1.6 |
| 21 | 0.6 | 0.2 | KOH 1.2 | (50%) | $(C_4H_9)_4NOH$ 0.003 | Benzene 60 | Same as above | | 91.3 | 28.0 | 21.5 | 49.5 | 1.3 |
| 22 | 0.6 | 0.2 | LiOH 1.2 | (50%) | $[(CH_3)_3NCH_2C_6H_5]OH$ 0.003 | Anisole 60 | 60 Chloroform added dropwise | 5.0 | 88.0 | 43.3 | 7.3 | 50.6 | 5.9 |

## Claims

1. A process for the preparation of salicylaldehyde and *p*-hydroxybenzaldehyde which comprises reacting an unsubstituted phenolic compound in aqueous alkali solution with chloroform in a heterogeneous system according to the Reimer-Tiemann reaction, in the presence of a phase transfer catalyst, characterised in that the phase transfer catalyst is a cationic-type phase transfer catalyst used in the presence of an aliphatic noncyclic ether as solvent or an amphoteric-type phase transfer catalyst used in the presence of an inert organic solvent.

2. A process as claimed in Claim 1, wherein the catalyst is a cationic-type phase transfer catalyst, being a quaternary ammonium salt-type phase transfer catalyst, a quaternary phosphonium salt-type phase transfer catalyst or a pyridinium salt-type phase transfer catalyst.

3. A process as claimed in Claim 2, wherein the catalyst is a quaternary ammonium or phosphonium salt-type phase transfer catalyst, being lauryltrimethylammonium chloride, cetyltrimethylammonium chloride, cetyltributylphosphonium bromide, triethylbenzylammonium chloride or tetrabutylammonium hydroxide.

4. A process as claimed in Claim 1, 2 or 3, wherein the catalyst is a cationic-type quaternary salt in which the anionic moiety thereof consists of a hydroxy group.

5. A process as claimed in Claim 1, wherein the catalyst is an amphoteric-type phase transfer catalyst, being a betaine-type phase transfer catalyst or an amino acid-type phase transfer catalyst.

6. A process as claimed in Claim 1, wherein the catalyst is an amphoteric-type phase transfer catalyst, being 1-carboxy-N-lauryl-N,N-dimethylmethaneaminium hydroxide inner salt or N,N-distearyl glycine.

7. A process as claimed in Claim 1, wherein the catalyst is an anionic-type phase transfer catalyst, being an alkali metal salt of a higher fatty acid, a higher alkylsulfonate or a salt of a sulfuric acid ester of a higher alcohol.

8. A process as claimed in any preceding Claim, wherein the phase transfer catalyst is used in an amount of from 0.1 to 20 mol % per mole of chloroform.

9. A process as claimed in Claim 1, 6, 7 or 8, wherein the solvent is an ether, an aromatic optionally halogenated hydrocarbon, an aliphatic alcohol, an ester or a ketone.

## Patentansprüche

1. Verfahren zur Herstellung von Salicylaldehyd und p-Hydroxybenzaldehyd durch Umsetzung einer nichtsubstituierten Phenolverbindung in einer wäßrigen alkalischen Lösung mit Chloroform in einem heterogenen System gemäß der Reimer-Tiemann-Reaktion in Gegenwart eines Phasentransferkatalysators, gekennzeichnet durch die Verwendung eines kationaktiven Phasentransferkatalysators in Gegenwart eines aliphatischen nichtcyclischen Ethers als Lösungsmittel oder eines ampholytischen Phasentransferkatalysators in Gegenwart eines inerten organischen Lösungsmittels.

2. Verfahren nach Anspruch 1, gekennzeichnet durch die Verwendung eines quartären Ammoniumsalzes, eines quartären Phosphoniumsalzes oder eines Pyridiniumsalzes also kationaktiven Phasentransferkatalysator.

3. Verfahren nach Anspruch 2, gekennzeichnet durch die Verwendung von Lauryltrimethylammoniumchlorid, Cetyltrimethylammoniumchlorid, Cetyltributylphosphoniumbromid, Triethylbenzylammoniumchlorid oder Tetrabutylammoniumhydroxid als quartären Ammonium- oder Phosphonium-Phasentransferkatalysator.

4. Verfahren nach Anspruch 1, 2 oder 3, gekennzeichnet durch die Verwendung eines kationaktiven quartären Salzes, in dem der anionische Teil eine Hydroxylgruppe ist, als Katalysator.

5. Verfahren nach Anspruch 1, gekennzeichnet durch die Verwendung eines Betains oder einer Aminosäure als ampholytischen Phasentransferkatalysators.

6. Verfahren nach Anspruch 1, gekennzeichnet durch die Verwendung eines inneren Salzes von 1-Carboxy-N-lauryl-N,N-dimethylmethanaminiumhydroxid oder von, N,N-Distearylglycin als ampholytischen Phasentransferkatalysator.

7. Verfahren nach Anspruch 1, gekennzeichnet durch die Verwendung eines Alkalimetallsalzes einer höheren Fettsäure, eines höheren Alkylsulfonats oder eines Schwefelsäureesters eines höheren Alkohols als anionaktiven Phasentransferkatalysator.

8. Verfahren nach einem der vorstehenden Ansprüche, gekennzeichnet durch die Verwendung des Phasentransferkatalysators in einer Menge von 0,1 bis 20 Molprozent je mol Chloroform.

9. Verfahren nach Anspruch 1, 6, 7 oder 8, gekennzeichnet durch die Verwendung eines Ethers, aromatischen ggf halogenierten Kohlenwasserstoffs, aliphatischen Alkohols, Esters oder Ketons als Lösungsmittel.

## Revendications

1. Procédé pour la préparation de salicylaldéhyde et de p-hydroxybenzaldéhyde, qui comprend la réaction d'un composé phénolyique non substitué dans une solution alcaline aqueuse avec du chloroforme dans un système hétérogène selon la réaction de Reimer-Tiemann, en présence d'un catalyseur de

transfert de phase, caractérisé par le fait que le catalyseur de transfert de phase est un catalyseur de transfert de phase de type cationique utilisé en présence d'un éther non cyclique aliphatique comme solvant ou d'un catalyseur de transfert de phase de type amphotérique utilisé en présence d'un solvant organique inerte.

2. Procédé selon la revendication 1, dans lequel le catalyseur de transfert de phase de type cationique est un catalyseur de transfert de phase de type sel d'ammonium, un catalyseur de transfert de phase de type sel de phosphonium quaternaire ou un catalyseur de transfert de phase de type sel de pyridinium.

3. Procédé selon la revendication 2, dans lequel le catalyseur de transfert de phase de type sel d'ammonium quaternaire ou de phosphonium est le chlorure de lauryltriméthylammonium, le chlorure de cétyltriméthylammonium, le bromure de cétyltributylphosphonium, le chlorure de triéthylbenzylammonium ou l'hydroxyde de tétrabutylammonium.

4. Procédé selon la revendication 1, 2 ou 3, dans lequel le catalyseur est un sel quaternaire de type cationique dans lequel le noyau anionique de celui-ci consiste en un groupe hydroxy.

5. Procédé selon la revendication 1, dans lequel le catalyseur de transfert de phase de type amphotérique est un catalyseur de transfert de phase de type bétaïne ou un catalyseur de transfert de phase de type amino-acide.

6. Procédé selon la revendication 1, dans lequel le catalyseur de transfert de phase de type amphotérique est un sel interne d'hydroxyde de 1-carboxy-N-lauryl-N,N-diméthyleméthaneammonium ou de la N,N-distéaryl-glycine.

7. Procédé selon la revendication 1, dans lequel le catalyseur de transfert de phase de type anionique est un sel de métal alcalin d'un acide gras supérieur, un alkylsulfonate supérieur ou un sel d'un ester d'acide sulfurique d'un alcool supérieur.

8. Procédé selon l'une des revendications précédentes, caractérisé par le fait que le catalyseur de transfert de phase est utilisé en une quantité de 0,1 à 20% mole par mole de chloroforme.

9. Procédé selon la revendication 1, 6, 7 ou 8, dans lequel le solvant est un éther, unhydrocarbure aromatique éventuellement halogéné, un alcool aliphatique, un ester ou une cétone.